# EUROPEAN PATENT SPECIFICATION

(11) **EP 0 402 963 B1**
(45) Date of publication and mention of the grant of the patent: **07.05.1997**
(21) Application number: 90115678.6
(22) Date of filing: 17.12.1984
(51) Int. Cl.: C07J 1/00

(54) **17-Alpha ethynyl steroids**
17-Alpha-Ethynyl Steroide
17-Alpha-éthynyl stéroides

(30) Priority: 05.01.1984 US 568558; 20.03.1984 US 591631; 20.04.1984 US 602300; 26.04.1984 US 604091
(43) Date of publication of application: 19.12.1990
(62) Divisional of application: 84308826.1
(73) Proprietor: PHARMACIA & UPJOHN COMPANY, Kalamazoo, Michigan 49001 (US)
(72) Inventor: Timko, Joseph Michael, c/o The Upjohn Company, Kalamazoo, Michigan 49001 (US); Vanrheenen, Verlan Henry, c/o The Upjohn Company, Kalamazoo, Michigan 49001 (US); Cha, Dae Yang, c/o The Upjohn Company, Kalamazoo, Michigan 49001 (US)
(74) Representative: Perry, Robert Edward

(56) References cited:
- EP-A- 0 104 054
- JOURNAL OF ORGANIC CHEMISTRY, vol. 43, no. 24, 24th November 1978, pages 4679- 4680, American Chemical Society, Washington, DC, US; G. NEEF et al.: "Revision of some 16-alkylated steroids"
- JOURNAL OF MEDICINAL CHEMISTRY, Vol. 11, no. 5, August 1968, pages 924-928
- JOURNAL OF ORGANIC CHEMISTRY, vol. 40, no. 15, 25th July 1975, pages 2250-2252

## Description

This invention relates to 17α-ethynyl steroids and to their use as intermediates in the preparation of, *inter alia,* betamethasone.

Neef *et al,* J. Org. Chem. 43(24):4679 (1978), describe *inter alia,* 17α-ethynyl-16β-methyl-5-androstene-3β,17β-diol.

Midland, J. Org. Chem. 40:2250 (1975), US-A-4055562 and US-A-4320236 disclose monolithium acetylide.

Novel compounds according to this invention are 17α-ethynyl-17β-hydroxy-16β-methylandrosta-1,4,9(11)-trien-3-one and 17α-ethynyl-17β-hydroxy-16β-methylandrosta-4,9(11)-dien-3-one. The triene can be used to prepare betamethasone, *via* the intermediate 17α,21-dihydroxy-16β-methylpregna-1,4,9(11)-triene-3,20-dione, by known procedures. The triene may be prepared by the reaction of 16β-methylandrosta-1,4,9(11)-triene-3,17-dione with monolithium acetylide at 0°C or less.

The monolithium acetylide can be prepared in different ways which is of importance because the temperature used in preparation is dependent on the method of preparation.

The monolithium acetylide can be prepared by bubbling acetylene through an ethereal solvent such as THF at about 20-25° until no further weight gain occurs (about 0.6 M in acetylene). An aliquot of this solution is cooled to about -60°C, and 4 equivalents (per equivalent of steroid) of an organo-lithium reagent such as n-butyllithium, methyllithium or phenyllithium are added with vigorous stirring to give a mixture which is about 0.57M in monolithium acetylide. Temperatures above about -10° and concentrations above about 0.8M in monolithium acetylide cause disproportionation to the insoluble dilithium acetylide. Therefore, slow addition of pre-cooled (-20° or less) organo-lithium reagent to very cold solution of acetylene in a dry solvent such as THF, diethyl ether, dioxane, and DME is preferred. It is preferred the temperature be kept at -80 to -25°, more preferably at -80 to -40°. If the organic solvent is at a temperature of -40° or warmer, the organo-lithium solution should be added slower using a higher stirring rate to facilitate heat transfer. The monolithium acetylide solution should be used immediately after preparing as letting it stand even at -78° for 6 hours may lower the yield 10%.

A stabilized monolithium acetylide is preferably used. It permits preparation of the monolithium acetylide at higher temperatures which is commercially advantageous. The stabilized monolithium acetylide is prepared in 3 alternative ways, two-pot (preferred), one-pot or in situ.

In the two-pot process, a sufficient quantity of acetylene is first dissolved in a suitable dry organic solvent. The temperature at which the acetylene can be dissolved in the dry organic solvent is not critical. The temperature affects the solubility and therefore the concentration of the acetylene. However, before the acetylene solution is contacted with the lithium complex it must be cooled to 0° or less. The nature of the organic solvent is not critical so long as it does not react with acetylene, organolithium compounds or amines. Suitable dry organic solvents include THF, dioxane, diethyl ether, t-butyl methyl ether and dimethoxyethane. The preferred solvent is THF. It is convenient for the organic solvent to be saturated with acetylene. Second, an organo-lithium compound is contacted with a stabilizing amine at 0° or less in a suitable dry organic solvent. Organo-lithium compounds include n-butyllithium, phenyllithium, and methyllithium, preferred is n-butyllithium. A stabilizing amine is an amine (primary, secondary or tertiary) which when reacted with an organo-lithium compound to form a lithium complex and/or corresponding lithium amide subsequently reacted with acetylene forms a stabilized monolithium acetylide which will not significantly disproportionate at 0° and below which is reactive towards 17-keto steroids. Stabilizing amines include N,N-diisopropyl ethyl amine, triethylamine, diisopropyl amine, t-butyl amine, diethylamine, dicyclohexylamine, hexamethyl disilazane, and pyrrolidine. Some amines such as pyrrolidine are only useful with some steroids such as 16β-methylandrosta-1,4,9(11)-triene-3,17-dione. Amines which are not operable include ethylenediamine and pyridine. Preferred is diisopropylamine or triethylamine. The same dry organic solvents useful to dissolve the acetylene in are also useful here. It is preferred that the same solvent or mixture of solvents be used for dissolving the acetylene in as for the reaction of the organo-lithium compound with the stabilizing amine. The reaction of the organo-lithium compound with a primary or secondary amine produces a lithium amide. A tertiary amine cannot produce a lithium amide and forms a complex, the nature of which is not known. Therefore, the reaction of an organo-lithium compound and a stabilizing amine will be considered to have produced a lithium complex. When the organo-lithium compound is added to the stabilizing amine it must be added slowly maintaining the temperature at about 25° or less. The reaction is complete in less than 30 min.

The third step of the two-pot process is contacting the lithium complex of step 2 with the acetylene solution of step 1 at 0° or less. It is preferred that the method of contacting be a slow addition of the lithium complex to the acetylene solution keeping the temperature at 0° or less. The reaction forming monolithium acetylide is complete in about 30 min.

In the two-pot process, the first two steps which are independent of each other can be performed in the reverse order. The organo-lithium compound can be reacted with the stabilizing amine prior to the dissolving of the acetylene in the dry organic solvent. All that is necessary is that both steps be independently performed and the temperature of both mixtures be 0° or less before they are combined in the third step.

The stabilized monolithium acetylide is reactive with aldehydes and ketones. It is preferred that the reactant be a 17-keto steroid. Commercially one of the most important uses of the ethynylation process is the transformation of 17-keto steroids to the corresponding 17α-ethynyl-17β-hydroxy steroid.

The monolithium acetylide produced by the process described herein is sufficiently stable at up to about 0° and is reactive with 16β-methyl 17-keto steroids. 16β-methyl-17-keto steroids are well known to those skilled in the art.

The 16-methyl-17-keto steroid to be ethynylated is added to the monolithium acetylide at 0° or less. The ethynylation reaction is complete in about 30 minutes. The 16-methyl-17-keto can be added as a solid, a slurry or as a solution. For convenience it is preferred that it be added as a solution. It should be added slowly as is known to those skilled in the art. Again, the same organic solvents used for steps 1 and 2 are useful for adding the material to be ethynylated to the monolithium acetylide. Again, it is preferable to use the same organic solvent as was used in steps 1 and 2. The ethynylated product is recovered by means well known to those skilled in the art.

In the one-pot process, the first step of dissolving the acetylene in a suitable dry organic solvent is the same as for the two-pot process. Second, the stabilizing amine is added to the acetylene solution at 0° or less. Third, the organo-lithium compound is added slowly to the mixture of the acetylene solution and the stabilizing amine again at 0° or less.

With the in situ process, the first step again is the same. Second, the 16-methyl-17-keto steroid to be ethynylated is added to the acetylene solution at 0° or less. Third, the organo-lithium compound and the stabilizing amine are reacted to form the lithium complex as in the second step of the two-pot process. Finally, the lithium complex is added slowly to the mixture of the acetylene solution containing the 16-methyl-17-keto steroid to be ethynylated at 0° or less and the monolithium acetylide is generated in situ.

While the above steps to prepare monolithium acetylide can be performed at 0° it is preferable to perform them at less than -20°, more preferable in a temperature range of about -20° to about -40°. Reducing the temperature reduces the amount of disproportionation, thereby increasing the amount of monolithium acetylide available for ethynylation.

Regardless of how the monolithium acetylide is prepared (two-pot, one-pot or in situ), when it is contacted with the Δ^{1,4}-3-keto 17-keto-16-methyl steroid at about -20° or less, the reaction time is very short, in the range of 5-60 min usually about 10-15 min.

The above discussion involves the temperatures at which the monolithium acetylide is prepared. The main advantage of using stabilized monolithium acetylide over non-stabilized monolithium acetylide is that it can be prepared and reacted at warmer temperatures. The reaction temperature of the monolithium acetylide with the 16β-methyl-17-keto steroid is usually about the same as the preparation temperature. If the reaction is performed above the decomposition temperature of the reagent, the yield will decrease. With the 16-methyl-Δ^{1,4}-3-keto steroids, the ethynylation reaction is performed at about -10° or less, preferably at about -40° or less, more preferably at about -70° or less.

No C₃ protecting group is necessary. However, it is preferable to add at least one equivalent of lithium ion prior to contacting the 17-keto steroid with the monolithium acetylide. The lithium ion helps keep the selectivity for the 17-ketone over the 3-ketone. The lithium ion is added as a lithium salt, for example, lithium chloride, lithium sulfate, etc. Preferred is lithium bromide or lithium perchlorate. Using the lithium ion permits the same yield and selectivity at -40° as was obtained without the lithium ion at -78°.

Generally, slightly more than one equivalent of monolithium acetylide per equivalent of 16-methyl-17-keto steroid is used. The more monolithium acetylide used, up to 4 equivalents, the better. Two equivalents are preferred and about four equivalents are more preferred.

When the acetylene addition is complete the excess acetylide is quenched or destroyed by reaction with a quenching agent which is any system such as water, saline or aqueous buffers depending on what final pH is desired. The quenching agent can be any system which protonates the acetylide, e.g. water, saline, buffers or non-aqueous systems containing protonating compounds, e.g. acetic acid, formic acid or other acid. Other quenching agents which are operable are those which will react with the acetylide such as acetone, methyl ethyl ketone or other ketones. The preferred quenching agent is acetone followed by aqueous acid. The 17α-ethynyl-17β-hydroxy-16-methyl steroids are obtained or isolated from the reaction mixture by means well known to those skilled in the art.

The reaction mixture is worked up by adding saline to the hydrolysis mixture which results in 2 phases. The organic phase is separated, washed, dried and concentrated to give the desired product. Alternatively, the product may be knocked out by water addition and recovered by known procedures.

The 17α-ethynyl-17β-hydroxy-16-methyl steroids are useful as intermediates in the preparation of 16-methyl-17α-hydroxyprogesterones and 16-methyl corticoids; see US-A-4041055. For example, 17α-ethynyl-17β-hydroxy-16β-methylandrosta-1,4,9(11)-trien-3-one is transformed to 17α,21-dihydroxy-16β-methylpregna-1,4,9(11)-triene-3,20-dione by the process of US-A-4041055. This Δ⁹⁽¹¹⁾-corticoid is transformed by known methods to the bromohydrin, 9β,11β-epoxide and finally to the corresponding 9α-fluoro-11β-hydroxy compound which is betamethasone (US-A-3485854).

In this specification:

All temperature are in degrees Centigrade.

TLC refers to thin-layer chromatography.

GLC refers to gas-liquid chromatography.

THF refers to tetrahydrofuran.

NMR refers to nuclear (proton) magnetic resonance spectroscopy; chemical shifts are reported in ppm (δ) downfield from tetramethylsilane.

LDA refers to lithium diisopropylamide.

Monolithium acetylate refers to LiC₂H and complexed forms thereof.

A stabilising amine is an amine (primary, secondary or tertiary), which when reacted with an organo lithium compound to form a lithium complex and subsequently reacted with acetylene forms a stabilised monolithium acetylide which will not significantly disproportionate at 0° and below and which is reactive towards 17-keto steroids. The following Examples illustrate the invention.

### Example 1 17α-Ethynyl-17β-hydroxy-16β-methylandrosta-1,4,9(11)-trien-3-one

THF (50 ml) and diisopropylamine (40 ml) are mixed and cooled to -20°. Slowly n-butyl lithium (1.6 M in hexane, 195 ml) is added with cooling, maintaining the temperature in the range of -22 to -18°. When addition is complete the lithium diisopropylamide is maintained at -20°.

THF (360 ml) is saturated with acetylene by bubbling acetylene thru the THF at -20 to -15°. The acetylene saturated THF is then cooled to -25°.

The lithium diisopropylamide prepared above is added dropwise to the acetylene maintaining the reaction temperature less than -10°. When the lithium diisopropyl amide addition is complete, the monolithium acetylide is cooled to less than -40°.

16β-Methylandrosta-1,4,9(11)-triene-3,17-dione (US Patent 3,010,958, 20 g) is added in THF (60 ml) to the lithium acetylide at -40°. When the reaction is complete, acetone (24 ml) is added. The mixture is warmed while bubbling nitrogen thru the slurry to remove excess acetylene. Water (25 ml) is slowly added. Water (105 ml) is then added more quickly creating two phases which are separated. The aqueous phase is washed with THF (50 ml); the organic phases (pH is 9) are combined, washed with aqueous sulfuric acid and concentrated to 350 ml. Heptane (200 ml) is added, the mixture heated to 80° and held for 30 min. The mixture is cooled slowly to 30° and then to 18° for one hr. The mixture is filtered, the solid washed with heptane (20 ml) and then dried under reduced pressure with heat to give the title compound.

### Example 2 17α-Ethynyl-17β-hydroxy-16β-methylandrosta-1,4,9(11)-trien-3-one (IIB)

THF (315 ml) and diisopropylamine (122 g) are mixed and cooled to -30°. n-Butyllithium (480 g) is added slowly to form LDA maintaining the temperature at -20 ± 1°.

THF (1.36 l) is cooled to -20° and acetylene (100 g) is dissolved in the THF using a nitrogen sweep (for safety reasons). The mixture is cooled to less than -25° and the LDA mixture is added slowly keeping the temperature less than -20°. The entire monolithium acetylide mixture is cooled to -45°.

16β-Methylandrosta-1,4,9(11)-triene-3,17-dione (70 g) is dissolved in THF (260 ml) and cooled to 20-22°.

The steroid mixture is added to the monolithium acetylide keeping the pot temperature at -40°, the addition is complete in 10 min or less. When the reaction is complete (about 10 min at -40°) the reaction mixture is quenched with acetone (106 ml) in water (35 ml) keeping the temperature at less than -35°. The mixture is sparged with nitrogen. Water (480 ml) is added and the mixture warmed to 45°. The mixture is degassed under reduced pressure for 30 min monitoring the acetylene by GLC and then cooled to about 25°.

The reaction mixture is worked up by means well known to those skilled in the art.

### Example 3 17α-Ethynyl-17β-hydroxy-16β-methylandrosta-4,9(11)-dien-3-one

A solution of THF saturated with acetylene is prepared by sparging acetylene into THF until no further weight gain occurs. To 112 ml of this solution, cooled to -60°, is added dropwise over 30 minutes n-butyllithium (1,6 M in hexane, 40 ml). The solution is warmed to -25° and then 3-hydroxy-16β-methylandrosta-3,5,9(11)-trien-17-one 3-methylether (5.0 g) is added. After 10 minutes, TLC indicates the reaction is complete, providing a solution containing 17α-ethynyl-3,17β-dihydroxy-16β-methylandrosta-3,5,9(11)-triene 3-methyl ester.

Degassed hydrochloric acid (6 N) is added to 17α-ethynyl-3,17β-dihydroxy-16β-methylandrosta-3,5,9(11)-triene 3-methyl ether until the pH equals and the solution is warmed to 0°. TLC analysis indicates the hydrolysis is complete after about one hour. The mixture is then poured into 5° water (120 ml). The THF is removed under reduced pressure and the aqueous solution extracted with methylene chloride. The organic phase is separated from the aqueous phase and the methylene chloride removed under reduced pressure to give a solid. The solid is triturated with cold methanol (5 ml, at 0° for 2 hours) to give title compound. mp 213-218°; NMR (CDCl₃) 0.83, 1.1, 1.33, 2.57, 5.57 and 5.73 ppm.

### Example 4 9β,11β-Epoxy-17α,21-dihydroxy-16β-methylpregn-4-ene-3,20-dione (US Patent 3,725,392)

Following the general procedure of US Patent 4,041,055 (Examples 37, 44, 51 and 66) and starting with 17α-ethynyl-17β-hydroxy-16β-methylandrosta-4,9(11)-dien-3-one (Example 3), 17α,21-dihydroxy-16β-methylpregna-4,9(11)-diene-3,20-dione is obtained which by methods well known to those skilled in the art is transformed (epoxidized) to give the title compound.

### Example 5 9α-Fluoro-11β,17α,21-trihydroxy-16β-methylpregna-1,4-diene-3,20-dione, betamethasone (US Patent 3,485,854)

Following the general procedure of US Patent 4,041,055 (Examples 37, 44, 51 and 66) and starting with 17α-ethynyl-17β-hydroxy-16β-methylandrosta-1,4,9(11)-trien-3-one (Example 1), 17α,21-dihydroxy-16β-methylpregna-1,4,9(11)-triene-3,20-dione is obtained which after reaction with HOBr, epoxidation and reaction with HF all by means well known to those skilled in the art produces the title compound.

## Claims

1. 17α-Ethynyl-17β-hydroxy-16β-methylandrosta-1,4,9(11)-trien-3-one.

2. Use of the compound of claim 1, for the preparation of 17α,21-dihydroxy-16β-methylpregna-1,4,9(11)-triene-3,20-dione, by known procedures.

3. Use of the compound of claim 1, for the manufacture of betamethasone, via the intermediate 17α,21-dihydroxy-16β-methylpregna-1,4,9(11)-triene-3,20-dione, by known procedures.

4. Use according to claim 3, wherein said intermediate is converted to betamethasone by reaction with HOBr, epoxidation and then reaction with HF.

5. Use according to any of claims 2 to 4, which additionally comprises preparing the compound of claim 1 by the reaction of 16β-methylandrosta-1,4,9(11)-triene-3,17-dione with monolithium acetylide at 0°C or less.

6. 17α-Ethynyl-17β-hydroxy-16β-methylandrosta-4,9(11)-dien-3-one.

## Patentansprüche

1. 17α-Ethynyl-17β-hydroxy-16β-methylandrosta-1,4,9(11)-trien-3-on.

2. Verwendung der Verbindung nach Anspruch 1 zur Herstellung von 17α,21-Dihydroxy-16β-methylpregna-1,4,9(11)-trien-3,20-dion nach bekannten Verfahren.

3. Verwendung der Verbindung nach Anspruch 1 zur Herstellung von Betamethason über das Zwischenprodukt 17α,21-Dihydroxy-16β-methylpregna-1,4,9(11)-trien-3,20-dion nach bekannten Verfahren.

4. Verwendung nach Anspruch 3, wobei das Zwischenprodukt durch Umsetzen mit HOBr, Epoxidieren und anschließende Reaktion mit Hf in Betamethason überführt wird.

5. Verwendung nach einem der Ansprüche 2 bis 4, wobei zusätzlich die Verbindung des Anspruchs 1 durch Umsetzen von 16β-Methylandrosta-1,4,9(11)-trien-3,17-dion mit Monolithiumacetylid bei 0°C oder darunter hergestellt wird.

6. 17α-Ethynyl-17β-hydroxy-16β-methylandrosta-4,9(11)-dien-3-on.

## Revendications

1. 17α-éthynyl-17β-hydroxy-16β-méthylandrosta-1,4,9(11)-triène-3-one.

2. Utilisation du composé suivant la revendication 1, pour la préparation de la 17α,21-dihydroxy-16β-méthylprégna-1,4,9(11)-triène-3,20-dione, par des modes opératoires connus.

3. Utilisation du composé suivant la revendication 1, pour la production de bêtaméthasone, en passant par l'intermédiaire consistant en 17α,21-dihydroxy-16β-méthylprégna-1,4,9(11)-triène-3,20-dione, par des modes opératoires connus.

4. Utilisation suivant la revendication 3, dans laquelle l'intermédiaire est transformé en bêtaméthasone par réaction avec HOBr, époxydation et ensuite réaction avec HF.

5. Utilisation suivant l'une quelconque des revendications 2 à 4, qui comprend en outre la préparation du composé suivant la revendication 1 par réaction de la 16β-méthylandrosta-1,4,9(11)-triène-3,17-dione avec l'acétylure de monolithium à une température égale ou inférieure à 0°C.

6. 17α-éthynyl-17β-hydroxy-16β-méthylandrosta-4,9(11)-diène-3-one.
